# EUROPEAN PATENT SPECIFICATION

(11) **EP 0 589 937 B1**
(45) Date of publication and mention of the grant of the patent: **15.03.2000**
(21) Application number: 92911644.0
(22) Date of filing: 17.06.1992
(51) Int. Cl.: A61K 38/00

(54) **USE OF 4-(4-CHLOROPHENYL-SULPHONYLCARBAMOYL) BENZOYL-L-VALYL-L-PROLINE 1(RS)-(1-TRIFLUOROACETYL-2-METHYLPROPYL) AMIDE FOR THE PREPARATION OF A MEDICAMENT FOR THE TREATMENT OF VASCULAR DISEASES**
VERWENDUNG VON 4-(4-CHLOROPHENYL-SULPHONYLCARBAMOYL) BENZOYL - L - VALYL - L - PROLINE 1(RS) - (1-TRIFLUOROACETYL-2-METHYLPROPYL)AMIDE ZUR HERSTELLUNG EINES MEDIKAMENTS FÜR DIE BEHANDLUNG VON GEFÄSSKRANKHEITEN
UTILISATION DE 4-(4-CHLOROPHENYL-SULFONYLCARBAMOYL)BENZOYL-L-VALYL-L-PROLINE 1(RS)-(1-TRIFLUORACETYL-2-METHYLPROPYL) AMIDE POUR LA PREPARATION D'UN MEDICAMENT POUR LE TRAITEMENT D'AFFECTIONS VASCULAIRES

(30) Priority: 18.06.1991 GB 91131649
(43) Date of publication of application: 06.04.1994
(73) Proprietor: ZENECA LIMITED, London W1Y 6LN (GB)
(72) Inventor: MEHTA, Jawehar Lal, UF College of Medicine,, Gainsville, FL 32610-0277 (US); SALDEEN, Tom Gustave Per, S-75236 Uppsala (SE); NICHOLS, Wilmer Wayne, Department of Medicine,, Univ. of Florida, Gainsville, FL 32610 (US)
(74) Representative: Giles, David Eric
(86) International application number: GB9201087
(87) International publication number: WO9222309

(56) References cited:
- EP-A- 0 189 305
- EP-A- 0 458 535
- EP-A- 0 458 536
- EP-A- 0 458 537
- EP-A- 0 463 811
- US-A- 4 910 190
- FASEB JOURNAL vol. 2, no. 4, 15 March 1988, BETHESDA, MD US page A346 KRELL R.D. ET AL 'Biochemical characterization of ICI 200880: a novel potent and selective inhibitor of human neutrophil elastase'
- AMERICAN HEART JOURNAL vol. 122, no. 5, November 1991, ST. LOUIS, USA pages 1245 - 1251 NICOLINI F.A. ET AL 'Leukocyte elastase inhibition and t-PA-induced coronary artery thrombolysis in dogs: beneficial effects on myocardial histology'

## Description

### Technical Field

The present invention concerns the use of the known compound 4-(4-chlorophenylsulphonylcarbamoyl)benzoyl-L-valyl-L-proline 1(RS)-(1-trifluoroacetyl-2-methylpropyl)amide, or a pharmaceutically acceptable salt thereof, in the preparation of a medicament for the treatment of acute myocardial ischaemia, angina and infarction.

### Background to Invention

The most frequent precipitating cause of acute myocardial ischaemia (unstable angina and myocardial infarction) is a thrombus occluding an atherosclerotic coronary artery. Early intravenous or intracoronary administration of thrombolytic agents, such as streptokinase or tissue plasminogen activator (t-PA), can reopen the occluded artery in approximately 75% to 85% of patients. However, a major problem associated with thrombolytic therapy is rethrombosis (and subsequent reocclusion) of the successfully recanalized artery, the rate of which has been reported to be as high as 30%. Failure to maintain coronary blood flew may result from continued in vivo platelet and neutrophil aggregation, the release of arachidonic acid metabolites which cause coronary spasm, or from capillary plugging by microemboli. In addition, there is evidence that reperfusion itself can lead to myocardial damage and detrimental effects on myocardial function mediated via reperfusion arrhythmias, depressed myocardial contractility and modulation of coronary vasomotor tone. At least part of these detrimental effects of reperfusion is related to activation of, and plugging by, neutrophils of the arterioles and capillaries that takes place during and following ischaemia. Reperfusion itself is often impaired ('no reflow' phenomenon) which itself contributes to tissue injury and thus recovery from ischaemia. The process of neutrophil trapping, activation, tissue damage and further activation and trapping of neutrophils, is believed to contribute to the progression of the underlying disease condition such as myocardial damage. Further, the loss of, or impairment to, the endothelium during neutrophil infiltration of tissues may also result in loss endothelium-derived relaxing factors. These latter phenomena may also potentiate rethrombosis and loss of coronary vascular reserve.

Nevertheless, the ingress into, and activation of, neutrophils in tissues following periods of ischaemia is believed to be involved in the natural scarring and healing processes and so, for adequate therapeutic or prophylactic benefit, a pharmaceutical agent needs to demonstrate the required balance of effects. There is thus a continuing clinical need to find a means to ameliorate injury in damaged or compromised vascular tissues such as reperfused tissues and thus effect a sustained treatment for vascular diseases and related conditions such as any one of those mentioned above. It has now been found that the therapeutic agent referred to above is useful in such treatment and this is a basis for the present invention.

### Disclosure of Invention

According to the present invention there is provided the use of 4-(4-chlorophenylsulphonylcarbamoyl)benzoyl-L-valyl-L-proline 1(RS)-(1-trifluoroacetyl-2-methylpropyl)amide, or a pharmaceutically acceptable salt thereof (hereinafter referred to as "the Agent"), in the manufacture of a medicament for use in the treatment of acute myocardial ischaemia, angina or myocardial infarction.

It should be noted that, although the active chemical entity is named here as "1(RS)" isomeric mixture, the invention also includes the use of a mixture containing any ratio of the "1(R)" and "1(S)" isomers of the above named entity, or the pharmaceutically acceptable salts thereof.

The active entity of the Agent, 4-(4-chlorophenylsulphonylcarbamoyl)benzoyl-L-valyl-L-proline 1(RS)-(1-trifluoroacetyl-2-methylpropyl)amide, and its production are described in US patent 4,910,190 where it was referred to as 3(RS)-[4-[(4-chlorophenyl)sulfonylamino-carbonyl)phenylcarbonyl]-L-valyl-N-[3-(1,1,1-trifluoro-4-methyl-2-oxopentyl)]-L-prolinamide, but the name given hereinabove is now preferred. It is noted that Dess-Martin periodinane, described as the preferred oxidant and used in the final step for the production of the compound in Examples 104 and 121 of said US patent, may in certain circumstances constitute an explosive hazard. Accordingly, it may be preferred to use an alternative oxidant for preparing the ketone from the corresponding alcohol. Alternative methods which may be useful include the use of oxalyl chloride, dimethyl sulfoxide and a tertiary amine (with the best results being obtained with 10-20 equivalents of oxidising agent); the use of acetic anhydride and dimethyl sulfoxide; the use of chromium trioxide pyridine complex in methylene chloride; and the use of alkaline potassium permanganate solution. For example, the compound may be obtained from the corresponding alcohol in approximately 60% yield using two equivalents of the latter oxidant. The compound may be formulated in conventional manner, for example, as described in the accompanying Example and then subsequently diluted to the required concentration with physiological saline just prior to its use.

As a yet further aspect of the invention, there is provided the use of the Agent as defined above wherein the medicament additionally contains one or more other agents indicated for use in treating said disease conditions. Such agents include those indicated to affect the rate or magnitude of reperfusion or to prevent reocclusion or, in the case of coronary artery disease, to prevent cardiac arrhythmia, fibrillation and/or myocardial damage. Such an agent may typically be, for example, a thrombolytic agent, an anticoagulant, an antithrombotic substance, a vasodilator, an antiarrhythmic or antifibrillatory agent, a thromboxane antagonist, an oxygen free-radical scavenger, or an inhibitor of transglutaminase activity, especially of Factor XIIIa activity.

Suitable thrombolytic agents include, for example, those of the type known as plasminogen activators, for example streptokinase, urokinase, prourokinase and tissue plasminogen activator (t-PA), or their derivatives, analogues or conjugates, or a mixture thereof, obtained from natural sources or by recombinant genetic engineering methodology. One particular thrombolytic agent of particular interest is t-PA.

Suitable anticoagulants include, for example, heparin, warfarin, aspirin, anisindione, phenidone and bishydroxycoumarin. Suitable antithrombic substance includes any substance which prevents or impedes the development of blood clots, including, for example, human activated protein C or an analogue thereof, as well as, for example, hirudin or an analogue or derivative, for example as noted in U.S. patent 4,944,943. Suitable vasodilators include, for example, a nitrate, papaverine, nicotinic acid and cyclandelate. Suitable antiarrhythmic or antifibrillatory agents include, for example, quinidine, procainamide, disopyramide, lidocaine, tocainide, phenyltoin, flecainide, encainide, amiodarone, bretylium, doflilium, verapamil, diltiazem, sotalol, propranolol, nadolol, metoprolol and 4-(N-ethyl-N-phenylamino)-1,2-dimethyl-6-(methylamino)pyrimidinium chloride.

Suitable oxygen free radical scavengers include, for example, superoxide dismutase, for example the copper/zinc form of cow or human origin, and mercapto-containing inhibitor of angiotensin converting enzyme (ACE), such as, for example, captopril, zofenopril, fentiapril or fosinopril. Suitable thromboxane antagonists include, for example, (4Z)-6-[[2RS,4RS,5SR]-2-(2-chlorophenyl)-4-(2-hydroxyphenyl)-1,3-dioxan-5-yl)]hex-4-enoic acid.

The individual therapeutically active agents may be administrable simultaneously, sequentially or separately, by the route and in the dosage which is well known in the art and which is customary for the particular active agents involved.

In use, the Agent will generally be administrable for prophylactic or therapeutic treatment in the form of a conventional pharmaceutical composition, for example, as generally described in US patent 4,910,190, and preferably as a sterile intravenous injection. The formulations may be made by conventional procedures well know in the art. An illustrative formulation providing a solution containing a concentration of 10 mg/ml of the Agent and suitable for use as an injectable solution is described in the accompanying Example.

In general, the Agent will be administrable to humans so that a daily dose in the general range, for example, 0.5 to 20 mg/kg (especially 3 to 7 mg/kg) of 4-(4-chlorophenylsulphonyl-carbamoyl)benzoyl-L-valyl-L-proline 1(RS)-(1-trifluoroacetyl-2-methylpropyl)amide, or an equivalent amount of a pharmaceutically acceptable salt thereof, is received given intravenously. However, it will be readily understood that it may be necessary to vary the dose of therapeutic product administered in accordance with well known medical practice to take account of the nature and severity of the disease or condition under treatment, any concurrent therapy, and of the age, weight and sex of the patient receiving treatment. In addition, it will be appreciated that in acute situations the Agent may be administered initially as a first bolus dose followed by smaller doses at regular intervals or by means of a continuous intravenous drip.

The utility of the Agent may be demonstrated in conventional therapeutic intervention trials, in which improvement in clinical or biochemical parameters may be measured. Thus, utility in the treatment of impaired cardiovascular function, for example, myocardial ischaemia, may be assessed by monitoring over a period of at least 2 weeks mortality, morbidity, cardiovascular haemodynamics (such as cardiac output and ejection fraction) and indices of the extent of myocardial damage such as the total release of creatine kinase and LDH.

In addition to clinical evaluation in man, the beneficial effects of the Agent may be demonstrated in laboratory animals in experimental paradigms well known in the art.

One such model of coronary arterial thrombosis is, for example, a dog in vivo reperfusion model involving electrically-induced coronary arterial thrombosis and subsequent thrombolysis by administration t-PA, according to the general method described by J L Mehta et al. (Thrombosis Research, 1990, 88, 13-21). The effect of the test substance is assessed by study of any of a variety of conventional parameters for assessing degree of myocardial ischaemia, for example by study of haemostatic parameters and histological study of the excised myocardium. In particular, the extent of leukocyte accumulation in the myocardium was determined in the ischaemic LAD and non-ischaemic circumflex (Cx) regions in a semiquantitative fashion using the method described by J L Mehta et al. (Am. J. Physiol. 1990, 258, H1402-H1408). This involves grading both the intensity and extent of leukocyte accumulation on a scale of zero to 4 with zero signifying essentially no leukocytes and scale 4 signifying extensive presence in the lumina of microvasculature and the vessel walls, adventitia and myocardial interstitium. By way of example, in this test model, the Agent defined hereinabove when administered as its sodium salt in phosphate buffered saline at the rate of 5mg/kg/hour typically results in a significantly lower amount of leukocyte accumulation without any toxic or other untoward effects being evident during the experiment or from the histological examination.

Another suitable model for evaluation of the Agent in coronary arterial thrombosis is, for example, the conscious dog in vivo model involving electrically-induced coronary arterial thrombosis and infarction according to the general method described by B R Lucchesi et al., (Thrombosis Research, 1980, 17, 841-853) or the modification described by D J Fitzgerald et al. (J. Clin. Invest., 1986, 77, 496-502).

Still further suitable models for evaluation of the Agent in arterial thrombosis are described in the articles by B R Ito et al. (Blood Cells, 1990, 16, 145-166) and B R Lucchesi and K M Mullane (Ann. Rev. Pharmacol. Toxicol., 1986, 26, 201-224).

The following Example illustrates a typical formulation of the Agent for use in the invention.

### Example

This example describes a formulation for 4-(4-chlorophenylsulphonylcarbamoyl)benzoyl-L-valyl-L-proline 1(RS)-(1-trifluoroacetyl-2-methylpropyl)amide (referred to as **COMPOUND**) which provides a strength of 10 mg/ml as the sodium salt in phosphate-buffered saline and which is suitable for an injectable solution. A corresponding placebo formulation is also provided. The prepared solutions are preferably sealed in ampoules of a convenient size, for example 5 ml, and stored with refrigeration until use.

| Ingredient | Weight per ml | |
|---|---|---|
| | 10.0 mg | Placebo |
| COMPOUND (Note 1) | 10.0 mg | -- |
| Dibasic sodium phosphate, heptahydrate, USP | 11.97 mg | 10.74 mg |
| Monobasic sodium phosphate, monohydrate, USP | 0.74 mg | 1.25 mg |
| Sodium chloride, USP | 4.50 mg | 5.48 mg |
| 1 N Sodium hydroxide solution or 0.05 M monobasic sodium phosphate solution (Note 2) | q.s. | q.s. |
| Water for Injection, USP q.s. ad | 1.0 ml (1.01 g) | 1.0 ml (1.01 g) |

| | | |
|---|---|---|
| NOTES: (1) The nominal concentration of COMPOUND in this formulation is 10 mg/ml. A manufacturing adjustment is made for the drug substance purity. | | |
| (2) Added to adjust pH to 7.0-7.5 | | |

### Manufacturing directions for the formulation of COMPOUND

1. Charge approximately 90% of the required amount of "Water for Injection, USP" to a vessel equipped with a suitable agitation device, and connected to a heater/cooler circulation bath.
2. Adjust the temperature of the circulation bath to 30 °C.
3. Charge with continuous stirring, the required amount of dibasic sodium phosphate, heptahydrate, USP and continue stirring until dissolved.
4. Charge very slowly with continuous stirring the required amount of COMPOUND.
5. Continue to stir for approximately 30 minutes until dissolved, then decrease the temperature of the circulation bath to 25 °C.
6. Charge with continuous stirring the required amount of monobasic sodium phosphate, monohydrate, USP and continue stirring until dissolved.
7. Charge with continuous stirring the required amount of sodium chloride, USP and continue stirring until dissolved.
8. Measure the pH and adjust to 7.0 to 7.5 with 1 N sodium hydroxide solution or 0.05 M monobasic sodium phosphate solution, if necessary.
9. Bring the batch to final weight (calculated from specific gravity of 1.01) with "Water for Injection, USP".
10. Aseptically filter the bulk solution into a suitable, sterilised filling vessel. Aseptically fill and seal the ampoules.
11. Leak test ampoules and visually inspect for particulate matter and other defects.

### Manufacturing directions for the placebo formulation

The procedure listed above is carried out with the omission of steps 2, 4 and 5, and without the need for temperature control.

## Claims

1. The use of 4-(4-chlorophenylsulphonylcarbamoyl)benzoyl-L-valyl-L-proline 1(RS)-(1-trifluoroacetyl-2-methylpropyl)amide, or a pharmaceutically acceptable salt thereof, in the manufacture of a medicament for use in the treatment of acute myocardial ischaemia, angina or myocardial infarction.

2. The use as claimed in claim 1 wherein the medicament is for the treatment of acute myocardial ischaemia.

3. The use as claimed in claim 1 wherein the medicament is for the treatment of angina.

4. The use as claimed in claim 1 wherein the medicament is for the treatment of myocardial infarction.

5. The use as claimed in any of claims 1 to 4 wherein the medicament additionally contains one or more other agents indicated for use in the treatment of acute myocardial ischaemia, angina or myocardial infarction.

6. The use as claimed in claim 5 wherein the medicament contains a thrombolytic agent, an anticoagulant, an antithrombotic substance, a vasodilator, an antiarrythmic agent, an antifibrillatory agent, a thromboxane antagonist, an oxygen free-radical scavenger or an inhibitor of transglutaminase activity,

## Patentansprüche

1. Verwendung von 4-(4-Chlorphenylsulfonylcarbamoyl)benzoyl-L-valyl-L-prolin-1(RS)-(1-trifluoracetyl-2-methylpropyl)amid oder eines pharmazeutisch verträglichen Salzes davon zur Herstellung eines Medikaments zur Verwendung bei der Behandlung von akuter Myokardischämie, Angina pectoris oder Myokardinfarkt.

2. Verwendung nach Anspruch 1, wobei das Medikament zur Behandlung von akuter Myokardischämie dient.

3. Verwendung nach Anspruch 1, wobei das Medikament zur Behandlung von Angina pectoris dient.

4. Verwendung nach Anspruch 1, wobei das Medikament zur Behandlung von Myokardinfarkt dient.

5. Verwendung nach einem der Ansprüche 1 bis 4, wobei das Medikament ferner ein oder mehrere weitere Mittel enthält, die zur Verwendung bei der Behandlung von akuter Myokardischämie, Angina pectoris oder Myokardinfarkt angezeigt sind.

6. Verwendung nach Anspruch 5, wobei das Medikament ein Thrombolytikum, ein Antikoagulantium, eine antithrombotische Substanz, einen Vasodilatator, ein Antiarrhythmikum, ein Antifibrillantium, einen Thromboxanantagonisten, einen Fänger für freie Sauerstoffradikale oder einen Inhibitor der Transglutaminaseaktivität enthält.

## Revendications

1. Utilisation de 4-(4-chlorophénylsulfonylcarbamoyl)benzoyl-L-valyl-L-proline 1(RS)-(1-trifluoroacétyl-2-méthylpropyl)amide ou d'un sel pharmaceutiquement acceptable de celui-ci, dans la fabrication d'un médicament en vue de l'emploi pour le traitement de l'ischémie myocardique aiguë, de l'angine ou de l'infarctus du myocarde.

2. Utilisation selon la revendication 1, dans laquelle le médicament est destiné au traitement de l'ischémie myocardique aiguë.

3. Utilisation selon la revendication 1, dans laquelle le médicament est destiné au traitement de l'angine.

4. Utilisation selon la revendication 1, dans laquelle le médicament est destiné au traitement de l'infarctus du myocarde.

5. Utilisation selon l'une quelconque des revendications 1 à 4, dans laquelle le médicament contient en plus un ou plusieurs autres agents indiqués pour l'emploi dans le traitement de l'ischémie myocardique aiguë, de l'angine ou de l'infarctus du myocarde.

6. Utilisation selon la revendication 5, dans laquelle le médicament contient un agent thrombolytique, un anticoagulant, une substance antithrombique, un vasodilatateur, un agent anti-arythmique, un agent antifibrillateur, un antagoniste de thromboxane, un épurateur de radicaux libres oxygène ou un inhibiteur de l'activité transglutaminase.
